# EUROPÄISCHE PATENTSCHRIFT

(11) **EP 0 578 121 B1**
(45) Veröffentlichungstag und Bekanntmachung des Hinweises auf die Patenterteilung: **09.04.1997**
(21) Anmeldenummer: 93110431.9
(22) Anmeldetag: 30.06.1993
(51) Int. Cl.: A61M 16/00, A61M 16/04

(54) **Verfahren zur Messung und Regelung des Druckes in der Dichtmanschette eines Trachealtubus**
Process for measuring and regulating the pressure of a tracheal cuff
Procédé de mesure et de régulation de la pression dans le ballonnet d'un tube trachéal

(30) Priorität: 07.07.1992 DE 4222220
(43) Veröffentlichungstag der Anmeldung: 12.01.1994
(73) Patentinhaber: ESW-EXTEL SYSTEMS WEDEL Gesellschaft für Ausrüstung mbH, D-22876 Wedel (DE)
(72) Erfinder: Pothmann, Werner, Dr., D-20249 Hamburg (DE); Poock-Haffmans, Peter, Dipl.-Ing., D-2000 Wedel (DE); Steinberg, Bastian, Dr., D-2000 Hamburg 73 (DE); Harms, Dirk, Prof. Dr., D-2000 Hamburg 56 (DE)

(56) Entgegenhaltungen:
- EP-A- 0 362 824
- EP-A- 0 366 524
- DE-U- 9 104 637
- US-A- 5 004 472

## Beschreibung

Die Erfindung bezieht sich auf ein Verfahren gemäß dem Oberbegriff von Anspruch 1 und auf eine Vorrichtung zur Durchführung des Verfahrens.

Es ist bekannt, zur Beatmung von Intensivpatienten endotracheale Tuben mit einer in der Luftröhre (Trachea) aufblasbaren Manschette (Cuff) zu verwenden. Hierbei erfüllt die Manschette zwei wesentliche Aufgaben, zum einen ermöglicht sie eine gasdichte Beatmung, zum anderen vermeidet sie die Aspiration von Flüssigkeit aus der Mundhöhle des Patienten in seine Atemwege. Um diese Aufgaben zu erfüllen, wird üblicherweise ein fixer Manschettendruck von 20-20 mbar gewählt.

In Fig. 1 ist eine in dem DE-GBM 91 04 637 beschriebene Cuff-Manschettenanordnung zum Abdichten einer Manschette 12 eines in die Luftröhre eines Patienten eingesetzten Tubus 10 dargestellt. Die Anordnung weist einen Druckluftspeicher 14, eine von dem Druckluftspeicher 14 zu der Manschette 12 führende Druckleitung 16, ein elektrisch ansteuerbares Zuluftventil 18, ein elektrisch ansteuerbares Abluftventil 20, einen den Druck in der Manschette 12 messenden Drucksensor 22 und eine Steuereinheit 24 auf. Der Druckluftspeicher 14 wird über ein Reduzierventil 26 von einer Druckluftquelle mit Druckluft gespeist, wobei das Reduzierventil auf einen Wert eingestellt ist, der deutlich obenhalb des Normaldrucks in der Cuff-Manschette, aber unterhalb des Druckes liegt, der zu einem Zerreißen der Cuff-Manschette führen könnte. Die Steuereinheit 24 mißt über den Drucksensor 22 den Druck in der Cuff-Manschette 10 und vergleicht diesen Ist-Druck mit einem von der Steuereinheit 24 errechneten Soll-Druck, wobei der Soll-Druck die Vorgeschichte des Druckverlaufs und den aktuellen Verlauf des Druckabfalls bzw. -anstiegs berücksichtigen sollte. Bei einem zu geringen Druck steuert die Einrichtung 24 das Zuluftventil 18 an, der Druck in der Cuff-Manschette 10 wird durch die hergestellte Verbindung mit dem Druckluftspeicher 14 erhöht. Überschreitet der Ist-Druck dagegen den Soll-Druck, wird das Abluftventil 20 angesteuert, die Cuff-Manschette 12 wird mit der Außenluft verbunden, der Druck in der Cuff-Manschette wird entsprechend gesenkt.

Der Erfindung liegt die Aufgabe zugrunde, ein Verfahren zur Messung und Regelung des Druckes in der Dichtmanschette eines Trachealtubus vorzuschlagen, durch welches auch bei großen und steilen, beispielsweise durch Hustenstöße des Patienten hervorgerufenen Druckanstiegen ein konstanter Andruck der Dichtmanschette an die Luftröhre des Patienten zwecks Erzielung einer gasdichten Beatmung und zwecks Verhinderung der Aspiration von Flüssigkeiten aus der Mundhöhle des Patienten in seine Atemwege gewährleistet wird.

Die Aufgabe wird erfindungsgemäß dadurch gelöst, daß von der Regeleinrichtung von dem Nominaldruck in der Dichtmanschette abweichende, große und steile Druckanstiege erkannt und als eine zu erwartende Weitstellung der Luftröhre und daraus resultierend als Abfall des Druckes in der Dichtmanschette gewertet werden, daß durch die Regeleinrichtung der in der Dichtmanschette herrschende Druck durch Luftzugabe für eine begrenzte und definierte Zeitdauer vorausschauend erhöht wird, und daß anschließend der in der Dichtmanschette vorhandene Druck mit einer kleineren Regelgeschwindigkeit gegen den ursprünglichen Nominaldruck geregelt wird.

Ausgestaltungen des erfindungsgemäßen Verfahrens sind in den Unteransprüchen 2 bis 6 beschrieben.

Eine Vorrichtung zur Durchführung des erfindungsgemäßen Verfahrens ist im Anspruch 7 beansprucht, während die Unteransprüche 8 bis 10 Weiterbildungen der erfindungsgemäßen Vorrichtung enthalten.

In der Zeichnung ist ein Ausführungsbeispiel nach der Erfindung dargestellt, und zwar zeigt:
- Fig. 2: eine erfindungsgemäße Vorrichtung in Blockschaltbildform, und
- Fig. 3: eine graphische Darstellung der Drücke in der Dichtmanschette in Abhängigkeit von der Zeit.

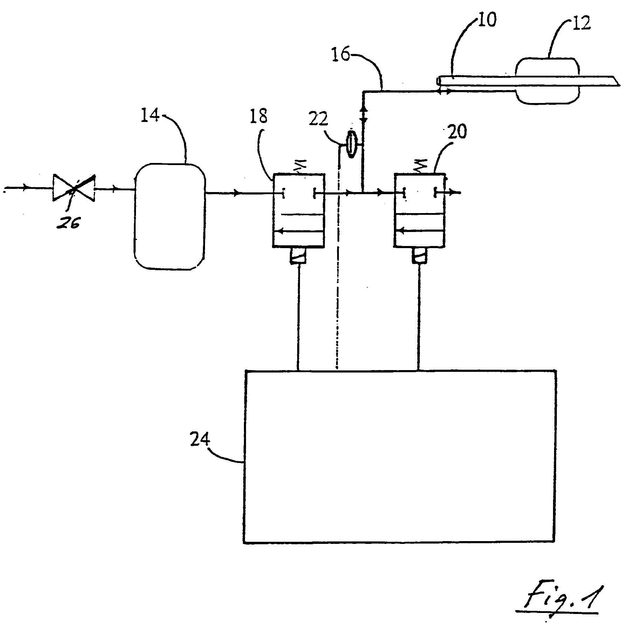

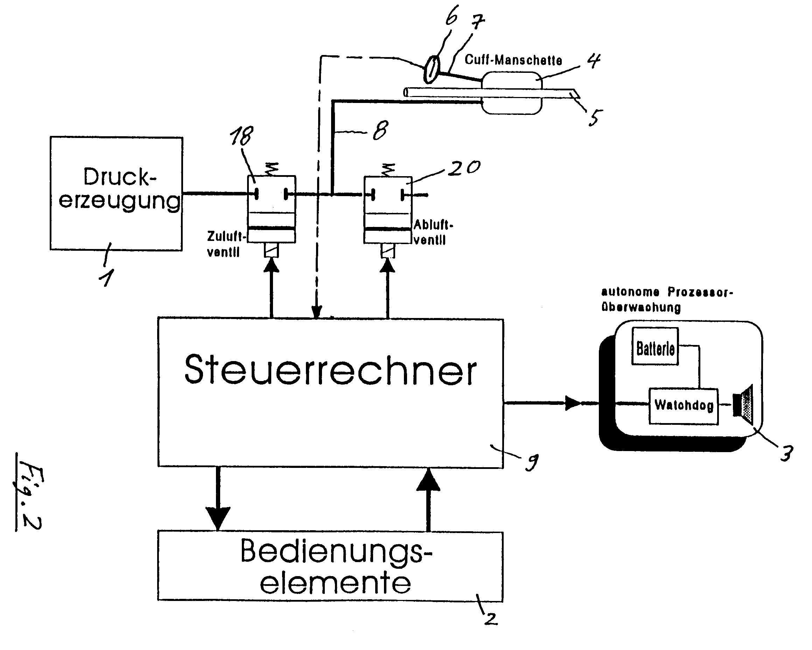

Die in Fig. 2 dargestellte diese Vorrichtung unterscheidet sich von der vorbekannten Cuff-Manschettenanordnung gemäß Fig. 1 insbesondere durch eine vereinfachte Darstellung der Einrichtung zur Druckerzeugung 1, durch dargestellte Bedienungselemente 2, durch eine autonome Prozessorüberwachungseinheit 3 und durch einen in unmittelbarer Nähe der Dichtungsmanschette 4 des Trachealtubus 5 angeordneten Drucksensor 6. Der Drucksensor 6 ist in einer zweiten Druckluftleitung 7 angeordnet, die neben der die Dichtmanschette 4 mit dem Zuluft- bzw. Abluftventil 18 bzw. 20 verbindende Druckluftleitung 8 an die Dichtmanschette 4 angeschlossen ist. Die Querschnitte der Druckluftleitungen 7 und 8 sind derart gewählt, daß ein optimales Regelverhalten erzielt wird. Andere Möglichkeiten bestehen beispielsweise darin, den Drucksensor in die Dichtmanschette zu integrieren bzw. in der Druckluftleitung 8 in unmittelbarer Nähe des Dichtmanschette anzuordnen. Durch die unmittelbare Nähe des Drucksensors 6 zur Dichtmanschette 4 wird vorteilhafterweise eine reale und schnelle Druckmessung erzielt.

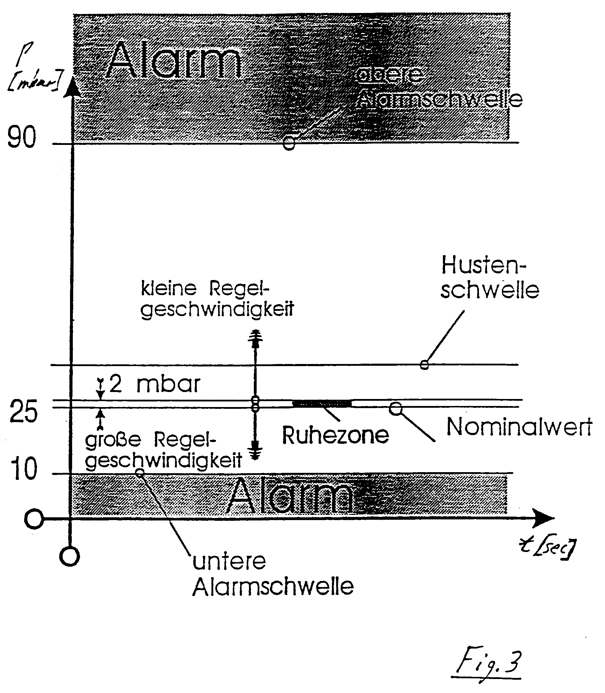

Unter Einbeziehung der graphischen Darstellung gemäß. Fig. 3 kann das erfindungsgemäße Verfahren zur Messung und Regelung des Druckes in der Dichtmanschette 4 eines Trachealtubus 5 wie folgt beschrieben werden.

Ein von dem Nominaldruck in der Dichtmanschette 4 abweichender großer und steiler Druckanstieg, der beispielsweise durch Hustenstöße hervorgerufen wird, wird von dem Drucksensor 6 gemessen und von der Regeleinrichtung als eine zu erwartende Weitstellung der Luftröhre und daraus resultierend als ein Abfall des Druckes in der Dichtmanschette 4 gewertet. Durch die Regeleinrichtung wird durch Luftzugabe der in der Dichtmanschette 4 herrschende Druck für eine begrenzte und definierte Zeitdauer vorausschauend erhöht. Anschließend wird der Dichtmanschettendruck mit einer kleineren Regelgeschwindigkeit gegen den ursprünglichen Nominaldruck geregelt.

Wie die obigen Ausführungen zeigen, führt das Unterschreiten eines erwünschten Druckwertes in der Dichtmanschette 4, kurz Nominaldruck genannt, zum unmittelbaren und schnellen Eingreifen der Regeleinrichtung 6, 9. Liegt der Momentandruck (Istwert) innerhalb einer definierten Ruhezone, greift die Regeleinrichtung 6, 9 nicht ein (Totzone). Steigt der Druck über die obere Schwelle der Ruhezone, greift die Regeleinrichtung 6, 9 mit kleiner Regelgeschwindigkeit ausgleichend ein.

Über- oder unterschreitet der Dichtmanschetten-Druck programmierbare Alarmschwellen, so wird ein Alarm ausgelöst.

Über die Bilanzierung zugeführter und abgeführter Luft werden Leckagen und Mikroleckagen erkannt und über Alarmsignale mitgeteilt.

Alle Regel- und Alarmschwellen sind über ein Benutzermenü einstellbar. Damit kann das Gerät den individuellen Bedürfnissen angepaßt werden. Neben dieser variablen Programmierung sind zwei vordefinierte Programmierungen abrufbar, und zwar für den Standardeinsatz bei Erwachsenen (Erwachsenenmenü und bei Kindern (Kindermenü).

## Patentansprüche

1. Verfahren zur Messung und Regelung des Druckes in der aufblasbaren Dichtmanschette (4) eines in die Luftröhre eines Patienten eingeführten Trachealtubus (5), vorzugsweise zur Erzielung eines konstanten Andruckes der Dichtmanschette (4) an die Luftröhre zwecks Erzielung einer gasdichten Beatmung des Patienten und zwecks Verhinderung der Aspiration von Flüssigkeiten aus der Mundhöhle des Patienten in seine Atemwege, unter Verwendung einer Regeleinrichtung (6,9), der der von einem Drucksensor (6) gemessene Istwert des in der Dichtmanschette (4) herrschenden Druckes zugeführt wird, dadurch gekennzeichnet, daß von der Regeleinrichtung (6, 9) von dem Nominaldruck in der Dichtmanschette (4) abweichende, große und steile Druckanstiege erkannt und als eine zu erwartende Weitstellung der Luftröhre und daraus resultierend als ein Abfall des Druckes in der Dichtmanschette (4) gewertet werden, daß durch die Regeleinrichtung (6, 9) der in der Dichtmanschette (4) herrschende Druck durch Luftzugabe für eine begrenzte und definierte Zeitdauer vorausschauend erhöht wird, und daß anschließend der in der Dichtmanschette (4) vorhandene Druck mit einer kleineren Regelgeschwindigkeit gegen den ursprünglichen Nominaldruck geregelt wird.

2. Verfahren nach Anspruch 1, dadurch gekennzeichnet, daß bei Über- oder Unterschreitung von Alarmdurckschwellen durch den Druck in der Dichtmanschette (4) ein Alarm ausgelöst wird.

3. Verfahren nach Anspurch 2, dadurch gekennzeichnet, daß die Alarmdruckschwellen programmierbar sind.

4. Verfahren nach Anspruch 1, 2 oder 3, dadurch gekennzeichnet, daß alle Regel- und Alarmschwellen über ein patientenabhängiges Benutzermenü eingestellt werden (variable Programmierung).

5. Verfahren nach Anspruch 1, 2 oder 3, dadurch gekennzeichnet, daß alle Regel- und Alarmschwellen über ein Standardmenü eingestellt werden.

6. Verfahren nach Anspruch 5, dadurch gekennzeichnet, daß ein Standardmenü für Erwachsene und ein Standardmenü für Kinder verwendet werden.

7. Vorrichtung zur Durchführung eines Verfahrens zur Messung und Regelung des Druckes in der aufblasbaren Dichtmanschette (4) eines in der Luftröhre eines Patienten eingeführten Trachealtubus (5), bestehend aus einer Einrichtung zur Drucklufterzeugung (1), einer zu der Manschette (4) führenden Druckluftleitung (8), einem elektrisch ansteuerbaren Zuluftventil (18), einem elektrisch ansteuerbaren Abluftventil (20), einem den Druck in der Manschette (4) messenden Drucksensor (6) und einer Steuereinheit,
dadurch gekennzeichnet,
daß von der Regeleinrichtung (6,9) von dem Nominaldruck in der Dichtmanschette (4) abweichende, große und steile Druckanstiege erkennbar und als eine zu erwartende Weitstellung der Luftröhre und daraus resultierend als ein Abfall des Druckes in der Dichtmanschette (4) wertbar sind,
daß durch die Regeleinrichtung (6, 9) der in der Dichtmanschette (4) herrschende Druck durch Luftzugabe für eine begrenzte und definierte Zeitdauer vorausschauend erhöhbar ist, und daß der in der Dichtmanschette (4) vorhandene Druck mit kleiner Regelgeschwindigkeit gegen den ursprünglichen Nominaldruck regelbar ist; und
daß der Drucksensor (6) in unmittelbarer Nähe der Dichtmanschette (4) angeordnet ist.

8. Vorrichtung nach Anspruch 7, dadurch gekennzeichnet, daß der Drucksensor in die Dichtmanschette (4) integriert ist.

9. Vorrichtung nach Anspruch 7, dadurch gekennzeichnet, daß neben der die Dichtmanschette (4) mit dem Zuluft- bzw. Abluftventil verbindenden Druckluftleitung (8) eine zweite Druckluftleitung (7) an die Dichtmanschette (4) angeschlossen ist, und daß die zweite Druckluftleitung (7) in unmittelbarer Nähe zur Dichtmanschette (4) einen Drucksensor (6) aufweist, dessen Meßsignale der Steuereinheit (9) zuführbar sind.

10. Vorrichtung nach Anspruch 7, 8 oder 9, dadurch gekennzeichnet, daß die Querschnitte der Druckluftleitungen (7, 8) derart gewählt sind, daß ein optimales Regelverhalten erzielt wird.

## Claims

1. Method of measuring and regulating the pressure in the inflatable sealing cuff (4) of a tracheal tube (5) introduced into the windpipe of a patient, preferably for attainment of a constant pressure of the sealing cuff (4) at the windpipe for the purpose of a gastight breathing of the patient and for the purpose of preventing aspiration of liquids from the oral cavity of the patient into his respiratory tracts, with use of a regulating equipment (6, 9), to which is fed the actual value, which is measured by a pressure sensor (6), of the pressure prevailing in the sealing cuff (4), characterised thereby that large and steep pressure rises deviating from the nominal pressure in the sealing cuff (4) are recognised by the regulating equipment (6, 9) and evaluated as a widening, which is to be expected, of the windpipe and resulting therefrom as a decay of the pressure in the sealing cuff (4), that the pressure prevailing in the sealing cuff (4) is increased in anticipation through addition of air for a limited and defined period of time by the regulating equipment (6, 9) and that subsequently the pressure present in the sealing cuff (4) is regulated at a lower regulating speed towards the original nominal pressure.

2. Method according to claim 1, characterised thereby that an alarm is triggered in the case of exceeding or falling below alarm pressure thresholds by the pressure in the sealing cuff (4).

3. Method according to claim 2, characterised thereby that the alarm pressure thresholds are programmable.

4. Method according to claim 1, 2 or 3, characterised thereby that all regulating and alarm thresholds are set by way of a patient-dependent user menu (variable programming).

5. Method according to claim 1, 2 or 3, characterised thereby that all regulating and alarm thresholds are set by way of a standard menu.

6. Method according to claim 5, characterised thereby that the standard menu is used for adults and a standard menu is used for children.

7. Device for carrying out a method for measuring and regulating the pressure in the inflatable sealing cuff (4) of a tracheal tube (5) introduced into the windpipe of a patient, consisting of an equipment for compressed air generation (1), a compressed air duct (8) leading to the cuff (4), an electrically controllable air feed valve (18), an electrically controllable air discharge valve (20), a pressure sensor (6) measuring the pressure in the cuff (4) and a control unit, characterised thereby that large and steep pressure rises deviating from the nominal pressure in the sealing cuff (4) are recognisable, and evaluatable as a widening, which is to be expected, of the windpipe and resulting therefrom as a decay of the pressure in the sealing cuff (4), by the regulating equipment (6, 9), that the pressure prevailing in the sealing cuff (4) can be increased in anticipation through addition of air for a limited and defined period of time by the regulating equipment (6, 9), and that the pressure present in the sealing cuff (4) can be regulated at low regulating speed towards the original nominal pressure; and that the pressure sensor (6) is arranged in the immediate proximity of the sealing cuff (4).

8. Device according to claim 7, characterised thereby that the pressure sensor is integrated into the sealing cuff (4).

9. Device according to claim 7, characterised thereby that apart from the compressed air duct (8) connecting the sealing cuff (4) with the air feed valve or air discharge valve, a second compressed air duct (7) is connected to the sealing cuff (4), and that the second compressed air duct (7) has in the immediate proximity of the sealing cuff (4) a pressure sensor (6), the measurement signals of which are feedable to the control unit (9).

10. Device according to claim 7, 8 or 9, characterised thereby that the cross-sections of the compressed air ducts (7, 8) are selected in such a manner that an optimal regulating behaviour is achieved.

## Revendications

1. Procédé pour la mesure et la régulation de la pression dans la garniture cylindrique d'étanchéité gonflable (4) d'un tube trachéal (5), introduit dans la trachée-artère d'un patient, de préférence pour obtenir une pression constante de la garniture cylindrique (4) contre la trachée-artère en vue d'obtenir une respiration étanche au gaz et artificielle du patient et en vue d'empêcher le patient d'aspirer des liquides depuis la cavité buccale par ses voies respiratoires, en utilisant un dispositif de réglage (6, 9) qui reçoit la valeur réelle, mesurée par un capteur de pression (6), de la pression régnant dans la garniture d'étanchéité (4), caractérisé en ce que le dispositif de réglage (6, 9) détecte des montées de pression rapides et importantes, qui s'écartent de la pression nominale dans la garniture d'étanchéité (4) et qui sont analysées en tant qu'élargissement prévisible de la trachée-artère et, à partir de là, en tant que chute de pression dans la garniture d'étanchéité (4), en ce que le dispositif de réglage (6, 9) augmente, de manière prévisionnelle, la pression régnant dans la garniture d'étanchéité (4) par apport d'air pendant une période de temps définie et limitée et en ce qu'ensuite, la pression présente dans la garniture d'étanchéité (4), est régulée avec une petite vitesse de réglage à l'encontre de la pression nominale d'origine.

2. Procédé selon la revendication 1, caractérisé en ce que, si la pression dans la garniture d'étanchéité (4) dépasse ou est inférieure à des seuils de pression d'alarme, une alarme se déclenche.

3. Procédé selon la revendication 2, caractérisé en ce que les seuils de pression d'alarme sont programmables.

4. Procédé selon la revendication 1, 2 ou 3, caractérisé en ce que les seuils de pression d'alarme et de réglage sont réglés par l'intermédiaire d'un menu utilisateur adapté au patient (programmation variable).

5. Procédé selon la revendication 1, 2 ou 3, caractérisé en ce que les seuils d'alarme et de réglage sont réglés par l'intermédiaire d'un menu standard.

6. Procédé selon la revendication 5, caractérisé en ce qu'un menu standard pour adulte et un menu standard pour enfant sont utilisés.

7. Dispositif pour la mise en oeuvre d'un procédé pour la mesure et le réglage de la pression dans la garniture d'étanchéité gonflable (4) d'un tube trachéal (5), introduit dans la trachée-artère d'un patient, se composant d'un dispositif de génération d'air comprimé (1), d'une conduite d'amenée (8) d'air comprimé à la garniture (4), d'une soupape d'air amené (18) commandée électriquement, d'une soupape d'air d'échappement (20) commandée électriquement, d'un capteur de pression (6) mesurant la pression dans la garniture (4) et d'une unité de commande,
caractérisé en ce que
le dispositif de réglage (6, 9) détecte des montées de pression rapides et importantes, qui s'écartent de la pression nominale dans la garniture d'étanchéité (4) et qui sont analysées en tant qu'élargissement prévisible de la trachée-artère et, à partir de là, en tant que chute de pression dans la garniture d'étanchéité (4),
en ce que le dispositif de réglage (6, 9) augmente, de manière prévisionnelle la pression régnant dans la garniture d'étanchéité (4) par apport d'air pendant une période de temps définie et limitée et en ce que la pression, présente dans la garniture d'étanchéité (4), est régulée avec une petite vitesse de réglage à l'encontre de la pression nominale d'origine, et
en ce que le capteur de pression (6) est disposé à proximité immédiate de la garniture d'étanchéité (4).

8. Dispositif selon la revendication 7, caractérisé en ce que le capteur de pression est intégré à la garniture d'étanchéité (4).

9. Dispositif selon la revendication 7, caractérisé en ce qu'une seconde conduite d'air comprimé (7) est raccordée à la garniture d'étanchéité (4) à côté de la conduite d'air comprimé (8) reliant la garniture d'étanchéité (4) à la soupape d'air amené et d'air d'échappement et en ce que la seconde conduite d'air comprimé (7) présente, à proximité immédiate de la garniture d'étanchéité (4), un capteur de pression (6) dont les signaux de mesure sont amenés à l'unité de commande (9).

10. Dispositif selon la revendication 7, 8 ou 9, caractérisé en ce que les sections transversales des conduites d'air comprimé (7, 8) sont choisies de manière à obtenir un comportement de réglage optimal.
